Europäisches Patentamt

European Patent Office

Office européen des brevets

(11) Publication number: **0 430 608 B1**

## EUROPEAN PATENT SPECIFICATION

(45) Date of publication of patent specification: **15.02.95**　(51) Int. Cl.⁶: **A61F 13/02**

(21) Application number: **90312811.4**

(22) Date of filing: **26.11.90**

(54) **Dressing including an indicator.**

<table>
<tr><td>

(30) Priority: **28.11.89 US 446341**

(43) Date of publication of application:
**05.06.91 Bulletin 91/23**

(45) Publication of the grant of the patent:
**15.02.95 Bulletin 95/07**

(84) Designated Contracting States:
**DE FR GB IT**

(56) References cited:
**US-A- 4 717 378**

</td><td>

(73) Proprietor: **E.R. Squibb & Sons, Inc.**
**Lawrenceville-Princeton Road**
**Princeton, N.J. 08540-4000 (US)**

(72) Inventor: **Flam, Eric**
**29 Ainsworth Avenue**
**East Brunswick,**
**New Jersey (US)**

(74) Representative: **Thomas, Roger Tamlyn et al**
**D. Young & Co.**
**21 New Fetter Lane**
**London EC4A 1DA (GB)**

</td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

The prevention and treatment of decubitus ulcers is an important aspect of health care. Patients who are comatose, diabetic, paraplegic or otherwise suffering from serious impairment of the neural and vascular systems are at risk of developing these pressure sores. Once the ulcer has progressed beyond the initial indications, treatment is difficult, long, and costly.

Studies have indicated correlations between thermal characteristics of skin and potential decubiti formation. These appear to be related to both increased temperatures and decreased blood flows to potential ulcer forming areas in compromised patients.

When an area of skin is compressed for a period of time the normal blood flow to the region is reduced. The amount of reduction depends on the applied pressure. The reaction of the tissues is a function of the pressure intensity, time of application and frequency of application. Under normal conditions, these factors produce a reaction of involuntary motion which tends to relieve the pressure from the affected region and redistribute it to another skin location. These motions occur with sufficient frequency so that no permanent tissue damage results. When the pressure is relieved, a hyperemic response occurs as the body seeks to compensate for the oxygen deprivation and metabolite accumulation by an increased blood flow. This response normally subsides in a few minutes as the tissues are restored to normal function. A component of this increased blood flow is a change in skin temperature during the response. Studies have shown that there is a pressure and time relationship for these events, and that the greater the applied pressure, the shorter the time interval of application must be if the effects are to be reversible.

This invention provides a dressing incorporating an indicator. The indicator conveys to the health care professional information about the condition of the patient's skin or wound beneath the dressing. The indicator eliminates the need for removing the dressing and having the health care professional perform a visual or other examination of the skin or wound. As a result, decisions about the course of treatment can be made in a faster and more cost effective manner.

More specifically, the invention provides a medical device comprising a dressing having a skin or wound contacting adhesive layer comprising one or more water soluble or swellable fluid interactive hydrocolloids dispersed in an elastomeric substance, and a backing film layer coextensive with said skin or wound contacting adhesive layer, characterised by an indicator means comprising a temperature sensing liquid crystal tape affixed to at least a portion of the surface of said backing film layer opposite the skin or wound contacting adhesive layer whereby when in use on the body information concerning the condition of the skin or wound beneath the dressing is available without the need to remove the dressing.

Particular reference will be made to the drawings which demonstrate preferred embodiments of the invention and in which:-

Figure 1 is a top view of an encapsulated temperature sensitive liquid crystal tape;

Figure 2 is a side view of the tape shown in Figure 1 in enlarged detail;

Figure 3 is a top view of a dressing incorporating the tape shown in Figures 1 and 2 as an indicator means;

Figure 4 is a side view of the dressing of Figure 3 in enlarged detail;

Figure 5 is a side view similar to Figure 4 of another dressing having an intermediate foam layer and also employing the tape shown in Figures 1 and 2 as an indicator means; and

Figure 6 is a perspective view showing the application of the temperature sensitive liquid crystal tape to a dressing which had been trimmed to fit onto the patient.

This invention provides dressing type products having one surface which contacts the skin or overlies a wound and an indicator incorporated within or at another surface of the dressing. The indicator conveys information to the health care professional about the condition of the underlying skin or wound.

The dressings can for example be fluid interactive hydrocolloid dressings capable of adhering to both dry and moist skin surfaces such as those described by Chen in U.S Patent US-A-3,339,546, by Chen et al. in U.S. Patent US-A-4,192,785, by Pawelchak et al. in U.S. Patent US-A-4,393,080, and by Doyle et al. in U.S. Patent US-A-4,551,490, and such hydrocolloid dressings including a polymeric foam layer for added flexibility and cushioning as described by Chen in U. S. Patent US-A-3,972,328 and by Pawelchak et al. in U. S. Patent US-A-4,538,603. The important criterion is that the dressing utilized transfers to the indicator information about the underlying skin or wound in an accurate and consistent manner.

The preferred indicator for incorporating within the dressing is a series of temperature sensitive, color responsive encapsulated liquid crystals. Such encapsulated liquid crystals are commercially available in the form of a tape having a pressure sensitive adhesive coated on one side. Suitable tapes are commercially available. For example, those available from Seven C's Incorporated under their trademark E-Z Temp®, from Davis Liquid Crystals, Inc. and from Eurand America, Inc. under their trademark Chameleon are suitable. These temperature sensing tapes have been applied directed to the forehead to measure temperature or have been applied to a flexible backing web for this purpose as shown by Flam in U.S. Patent US-A-3,661,142.

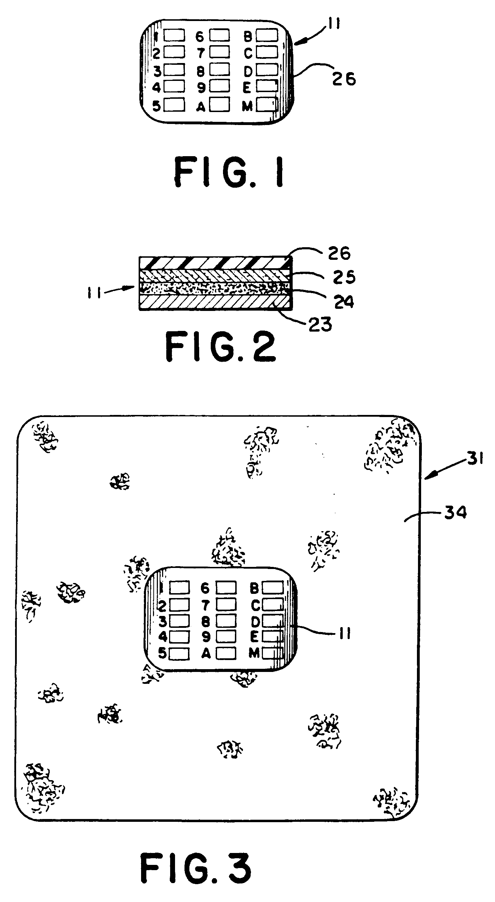

In the preferred embodiment of this invention as shown in the Figures, the encapsulated temperature sensitive liquid crystals are employed as a tape having a plurality of boxes with accompanying indicia in form of numerals and/or letters. The tape 11 as shown in Figure 1 consists of an array of 15 boxes where each box would represent the following temperature:

| Box | Temperature Legend (°C) |
|-----|--------------------------|
| 1 | 29° |
| 2 | 30° |
| 3 | 31° |
| 4 | 31.5° |
| 5 | 32 |
| 6 | 32.5 |
| 7 | 33 |
| 8 | 33.5 |
| 9 | 34 |
| A | 34.5 |
| B | 35 |
| C | 35.5 |
| D | 36 |
| E | 37 |
| M | 38 |

Of course, a tape can be employed with more or less than the 15 boxes shown and the rectangular boxes can be replaced by circles or other geometric patterns.

The encapsulated temperature sensitive liquid crystal tape 11 as best shown in Figure 2 consists of the liquid crystal substrate layer 25, a pressure sensitive acrylic type adhesive layer 24, an outer protective polymeric layer 26 of polyethylene terephthalate or other suitable material, and a release liner 23 such as silicone coated paper on the exposed surface of adhesive layer 24. Substrate layer 25 is visible through protective layer 26 as a black background with white indicia. When the temperature being sensed equals or is greater than the temperature range of one or more boxes, those boxes appear colored with green or blue green as the most prominent color. When the temperature being sensed is below that of a particular box, that box will not be visible against the black background. Similarly, when the temperature being sensed is substantially greater than the range of a particular box, that box will no longer be visible against the black background. Of course, other color schemes can be employed.

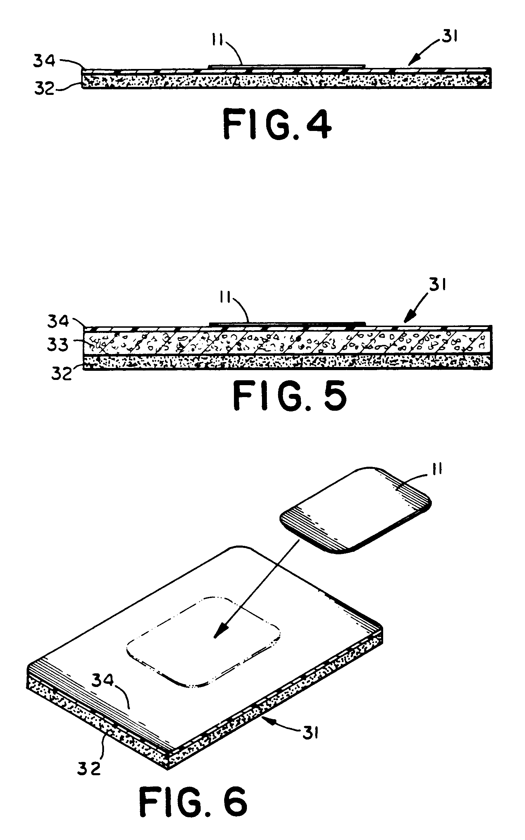

Tape 11 with release liner 23 removed is typically about 1 to 15 mils (25.4 to 381 $\mu$m) in thickness. Tape 11 is adhered by means of adhesive layer 24 to the top surface of a dressing. As shown in Figures 3 and 4, dressing 31 preferably consists of a fluid interactive hydrocolloid containing adhesive layer 32 and a backing film 34. As shown in Figure 5, dressing 31 can include a layer of polymeric foam 33 between adhesive layer 32 and backing film 34. The surface of foam layer 33 which contacts fluid interactive hydrocolloid adhesive layer 32 can be coated with a pressure sensitive adhesive to increase the strength of the bond. Of course, the bottom of adhesive layer 32 would be covered with release paper prior to use.

Adhesive layer 32 is formulated by blending one or more water soluble or water swellable hydrocolloids with an elastomeric substance. Suitable hydrocolloids include sodium carboxymethylcellulose, pectin, gelatin, guar gum, locust bean gum, gum karaya, and mixtures thereof. Suitable elastomeric substances include polyisobutylene, mixtures of polyisobutylenes, or mixtures of one or more polyisobutylenes and one or more non-acrylic elastomers such as butyl rubber and styrene radial or block copolymers such as styrene-butadiene-styrene (S-B-S) and styrene-isoprene-styrene (S-I-S) block type copolymers. Other materials can be included within the fluid interactive hydrocolloid adhesive layer such as mineral oil, tackifiers, antioxidants, cohesive strengthening agents such as water-insoluble cross-linked sodium carboxymethylcellulose and water-insoluble cross-linked dextran, and pharmaceutically active materials such as antibiotics, antiseptics, antiinflammatory agents, or materials having wound healing or skin soothing properties.

The dressings shown in Figures 3 to 5 are suitable for use on intact skin which may be susceptible to decubitus ulcer formation or over already formed and exudating ulcers. For example, a 10 mil (254 $\mu$m)

3

thick version of the dressing shown in Figures 3 and 4 would be suitable for use in a prophylactic manner on a patient who does not exhibit signs of ulcer formation but who because of illness and/or lack of mobility is considered to be at risk. The indicator permits the health care professional to monitor the condition of the patient's skin. An increase in temperature noted, for example, by a shift from box number 4 to box number 9 or one of the later boxes would signal that a change in treatment is needed. This could alert the health care professional to the need to increase blood circulation such as by massage, shifting the position of the patient more often, the use of a water-bed or other pressure distributing support system, or perhaps replacing the 10 mil (254 µm) thick dressing with a thicker version such as 20 mil (508 µm) or employing the more cushioned foam type dressing as shown in Figure 5.

Monitoring changes in temperature beneath the dressing can also be useful in determining the stage of an underlying pressure sore. An increase in temperature is indicative of a shift from stage I (skin is red) to a stage II (skin is blistered) or stage III (skin is broken) condition. By being aware of such a shift, the health care professional is able to administer appropriate treatment.

Analogously, the indicator dressing of this invention can be employed to measure healing of a pressure sore. In this instance, the patient may be started with the thicker foam containing dressing of Figure 5 and then by monitoring the healing the patient would be shifted to the thinner dressings of Figures 3 and 4.

Monitoring changes in temperature by means of the dressing and indicator device of this invention conveys other useful information such as the presence of infection associated with an increase in temperature or the presence of necrosis associated with a decrease in temperature. Again, the health care professional would be alerted to take appropriate medical procedures such as administering antibiotics if infection is confirmed or employing debriding agents to remove necrotic tissue. Other applications for the dressing and indicator device includes monitoring the hydration and/or dehydration level of the dressing and/or underlying wound since the temperature reading changes as a function of the thermal conductivity of the dressing.

The indicator can be attached directly to the top surface of the dressing during the manufacturing step. Preferably, the dressing and indicator are packaged separately until use. This is a particularly desired case where the indicator measures temperature and the dressing is subjected to sterilization. As shown in Figure 6, the health care professional is able to trim the dressing to the desired size and then remove release liner 23 from indicator tape 11 and adhere tape 11 to backing film 34 so as to be positioned directly over the area of the patient's body to be monitored.

The following examples are illustrative of the invention.

Example 1

A wound dressing was prepared as follows:
An adhesive layer was prepared consisting of

| Ingredient | Weight Percent |
|---|---|
| Polyisobutylene (Vistanex® LM-MH) | 8.0 |
| Butyl rubber (065) | 16.25 |
| Styrene-isoprene-styrene block copolymer (Kraton® 1107) | 6.0 |
| Tackifier (Pentalyn® H) | 12.75 |
| Mineral Oil | 11.5 |
| Pectin | 15.0 |
| Gelatin | 15.0 |
| Sodium carboxymethylcellulose | 15.0 |
| Antioxidant (Irganox® 1010) | 0.5 |

The hydrocolloid containing adhesive composition of the above formula was compounded by first forming a preblend of the polyisobutylene, butyl rubber, styrene-isoprene-styrene block copolymer, and antioxidant in a Sigma blade kneader extruder while heating the mass at about 150-155°C. The mineral oil was then added with continued agitation followed by the addition of the tackifier, temperature during this step was kept at about 120°C. Finally, the gelatin, pectin, and carboxymethylcellulose were added as powders while the mass was mixed and cooled. The resulting homogeneous mass was extruded to a thickness of about 10 mils (254 µm) and a 1 mil (25.4 µm) backing film of a polyether polyurethane was applied to one surface and a sheet of silicone coated release paper to the other surface.

4

The resulting dressing was die cut to a desired shape such as a 4 by 4 inch (2.54 cm by 2.54 cm) square, packaged, and sterilized.

A liquid crystal patch having a plurality of boxes capable of sensing temperatures from 29°C to 38°C coated with a layer of pressure sensitive adhesive was packaged in a carton with the sterilized dressing. Such liquid crystal patches are commercially available from Seven C's Incorporated.

Prior to use, the dressing is removed from the package and trimmed to the desired shape. Release liner 23 is removed and tape 11 is secured to dressing by pressing adhesive layer 24 onto backing film 34.

Example 2

A wound dressing was prepared as described in Example 1 except the adhesive mass was extruded to a thickness of about 20 mils (508 $\mu$m).

Example 3

A temperature sensing wound dressing of the construction shown in Figure 5 was prepared. The dressing includes a hydrocolloid containing adhesive mass of the formula described in Example 1 extruded to a thickness of about 50 mils (1270 $\mu$m). The foam layer employed was an open cell polyurethane foam, polyester type, of about 60 mils (1524 $\mu$m) thickness having a polyurethane skin on one surface and a pressure sensitive acrylic adhesive on the other surface. The pressure sensitive acrylic adhesive was pressed into contact with the fluid interactive hydrocolloid containing adhesive layer. At the time of use, a liquid crystal temperature indicator was secured to the top surface of the wound dressing.

Example 4

The following in vitro experiment was performed to compare temperature measurements on a heated surface measured through the dressing-type of Examples 1 to 3 with the temperature sensing tape applied directly to the surface.

A heated surface was covered with a 1/2 inch (12.7 mm) thick aluminium plate which in turn was covered with a 1 inch (2.54 cm) thick aluminium plate. Surface temperature of the top plate was measured with a calibrated Thermistor probe. Four samples of each of the dressings of Examples 1 to 3 with the liquid crystal tape applied to top surface were located across the 1 inch thick plate along with four samples of the liquid crystal tape applied directly by its adhesive layer 24.

The indicators were read and the corresponding temperatures were recorded. If one box was visible its corresponding temperature was recorded, if two boxes were visible then the average temperature was recorded.

The temperature of the aluminium plate was increased and after equilibration the indicators were again read. This was repeated until the maximum temperatures of the indicators was reached.

The following table lists the average of the temperature displayed by the four samples and includes the calculated standard deviation in parenthesis at each measurement.

| Temperature Measured With Probe (°F) – (°C) | Tape Applied Directly | Tape On Dressing of Example 1 | Tape On Dressing of Example 2 | Tape On Dressing Of Example 3 |
|---|---|---|---|---|
| 83.9 – 28.8 | 84.4 (0) – 29.1 | 84.4 (0) – 29.1 | 84.4 (0) – 29.1 | |
| 85.6 – 29.8 | 85.2 (0) – 29.6 | 85.2 (0) – 29.6 | 85.2 (0) – 29.6 | 84.2 (0) – 29.0 |
| 86.2 – 30.1 | 86.0 (0) – 30.0 | 86.0 (0) – 30.0 | 86.0 (0) – 30.0 | 85.2 (0) – 29.6 |
| 87.4 – 30.8 | 88.0 (0) – 31.1 | 87.8 (0.4) – 31.0 | 87.8 (0.4) – 31.0 | 86.0 (0.9) – 30.0 |
| 88.7 – 31.5 | 89.2 (0.2) – 31.8 | 89.1 (0) – 31.7 | 89.1 (0) – 31.7 | 86.7 (0) – 30.4 |
| 90.0 – 32.2 | 89.8 (0) – 32.1 | 89.7 (0.2) – 32.1 | 89.7 (0.2) – 32.1 | 88.0 (0) – 31.1 |
| 91.1 – 32.8 | 91.6 (0) – 33.1 | 91.6 (0) – 33.1 | 91.6 (0) – 33.1 | 89.1 (0) – 31.7 |
| 93.0 – 33.9 | 92.4 (0) – 33.6 | 92.4 (0) – 33.6 | 92.4 (0) – 33.6 | 89.8 (0) – 32.1 |
| 95.5 – 35.3 | 95.6 (0.3) – 35.3 | 95.3 (0.1) – 35.2 | 95.2 (0) – 35.1 | 91.3 (0.6) – 32.9 |

EP 0 430 608 B1

| Temperature Measured With Probe (°F)-(°C) | Tape Applied Directly | Tape On Dressing of Example 1 | Tape On Dressing of Example 2 | Tape On Dressing Of Example 3 |
|---|---|---|---|---|
| 96.5 ~35.8 | 96.0 (0) ~35.6 | 96.0 (0) ~35.6 | 96.0 (0) ~35.6 | 92.4 (0) ~33.6 |
| 97.6 ~36.4 | 97.1 (0.3)~36.2 | 97.1 (0.3)~36.2 | 97.1 (0.3)~36.2 | 93.2 (0) ~34.0 |
| 98.9 ~37.2 | 97.5 (0) ~36.4 | 97.5 (0) ~36.4 | 97.5 (0)~36.4 | 95.0 (0.2)~35.0 |
| 100.1 ~37.8 | 99.3 (0) ~37.4 | 99.3 (0) ~37.4 | 99.3 (0) ~37.4 | 95.5 (0) ~35.3 |
| 101.5 ~38.6 | 99.3 (0) ~37.4 | 99.3 (0) ~37.4 | 99.3 (0) ~37.4 | 96.0 (0.5)~35.6 |
| 101.7 ~38.7 | | | | 96.6 (0.5)~35.9 |

As will be seen, the readings made by the tape on top of the 10 mil (254 $\mu$m) and 20 mil (508 $\mu$m) dressing of Examples 1 and 2 were essentially identical to the readings made by the tape itself. The readings on top of the thicker dressing of Example 3 were offset in linear manner and still showed little standard derivation.

Thus, all three dressings with the indicator tape would convey in an accurate manner to the health care professional changes in temperature in the skin or a wound beneath each of the dressings.

**Claims**

1. A medical device comprising a dressing (31) having a skin or wound contacting adhesive layer (32) comprising one or more water soluble or swellable fluid interactive hydrocolloids dispersed in an elastomeric substance, and a backing film layer (34) coextensive with said skin or wound contacting adhesive layer (32), characterised by an indicator means comprising a temperature sensing liquid crystal tape (11) affixed to at least a portion of the surface of said backing film layer (34) opposite the skin or wound contacting adhesive layer (32) whereby when in use on the body information concerning the condition of the skin or wound beneath the dressing (31) is available without the need to remove the dressing (31).

2. The device of claim 1 wherein said water soluble or swellable hydrocolloids are selected from sodium carboxymethylcellulose, pectin, gelatin, guar gum, locust bean gum, gum karaya, and mixtures thereof, and said elastomeric substance is selected from polyisobutylene, mixtures of polyisobutylenes, and mixtures of one or more polyisobutylenes and one or more elastomers selected from butyl rubber and styrene radial or block copolymers.

3. The device of claim 2 wherein said fluid interactive hydrocolloid containing adhesive layer (32) comprises on a weight percentage basis about 8% polyisobutylene, about 16.25% butyl rubber, about 6% styrene-isoprene-styrene block copolymer, about 12.75% tackifier, about 11.5% mineral oil, about 15% sodium carboxymethylcellulose, about 15% pectin, about 15% gelatin, and about 0.5% antioxidant.

4. The device of claim 3 wherein said dressing backing film layer (34) is affixed directly to said fluid interactive hydrocolloid containing adhesive layer (32) and is a polyether polyurethane film.

5. The device of claim 4 wherein said fluid interactive hydrocolloid containing adhesive layer (32) is about 10 mils (254 $\mu$m) thick, said backing film layer (34) is about 1 mils (25.4 $\mu$m) thick, and said temperature sensing liquid crystal tape (11) is about 1 to 15 mils (25.4 to 381 $\mu$m) thick.

6. The device of claim 4 wherein said fluid interactive hydrocolloid containing adhesive layer (32) is about 20 mils (508 $\mu$m) thick, said backing film layer (34) is about 1 mils (25.4 $\mu$m) thick, and said temperature sensing liquid crystal tape (11) is about 1 to 15 mils (25.4 to 381 $\mu$m) thick.

7. The device of claim 3 wherein one surface of a polymeric foam layer (33) is affixed to said fluid interactive hydrocolloid containing adhesive layer (32), said foam layer having a skin or backing layer (34) on its opposite surface.

8. The device of claim 7 wherein said fluid interactive hydrocolloid containing adhesive layer (32) is about 50 mils (1270 $\mu$m) thick, said polymeric foam (33) is an open cell polyester polyurethane foam of about 60 mils (1524 $\mu$m) thick having a polyurethane skin, and said temperature sensing liquid crystal tape (11) is attached to said polyurethane skin and is about 1 to 15 mils (25.4 to 381 $\mu$m) thick.

9. The device of any preceding claim wherein said temperature sensing liquid crystal tape (11) has a plurality of indicia representative of different ranges of temperature.

**Patentansprüche**

1. Medizinische Vorrichtung, umfassend einen Wundverband (31) mit haut- oder wundberührender Klebeschicht (32), umfassend ein oder mehrere wasserlösliche oder mit Wasser quellfähige, mit Flüssigkeiten wechselwirkende, in einer elastomeren Substanz dispergierte Hydrokolloid(e) und eine mit der haut- oder wundberührenden Klebeschicht (32) ausdehnungsgleichen Trägerfilmschicht (34), gekennzeichnet durch eine Anzeigevorrichtung, umfassend ein temperaturempfindliches, an mindestens einem Teil der Oberfläche der Trägerfilmschicht (34) gegenüber der haut- oder wundberührenden Klebeschicht (32) befestigtes Flüssigkristallband (11), bei dem in Benutzung die Körperinformationen betreffend den Zustand der Haut oder der Wunde unter dem Wundverband (31) ohne die Notwendigkeit zur Entfernung des Wundverbands (31) zugänglich ist.

2. Vorrichtung gemäß Anspruch 1, bei der die wasserlöslichen oder mit Wasser quellbaren Hydrokolloide aus Natriumcarboxymethylcellulose, Pectin, Gelatine, Guaran, Johannisbrotgummi, Karayagummi und Gemischen daraus ausgewählt ist, und die elastomere Substanz aus Polyisobutylen, Gemischen aus Polyisobutylenen, und Gemischen eines oder mehrerer Polyisobutylene mit einem oder mehrerer Elastomere, die aus Butylgummi, und radialen oder Blockcopolymeren aus Styrol ausgewählt sind.

3. Vorrichtung gemäß Anspruch 2, bei der die mit Flüssigkeiten wechselwirkende, hydrokolloidhaltige Klebeschicht (32) auf Gewichtsprozentbasis etwa 8% Polyisobutylen, etwa 16,25% Butylgummi, etwa 6% Styrol-Isopren-Styrol Blockcopolymer, etwa 12,75% Klebrigmacher, etwa 11,5% Mineralöl, etwa 15% Natriumcarboxymethylcellulose, etwa 15% Pectin, etwa 15% Gelatine und etwa 0,5% Antioxidans umfaßt.

4. Vorrichtung gemäß Anspruch 3, bei der die Trägerfilmschicht (34) des Wundverbands direkt an der mit Flüssigkeiten wechselwirkenden, hydrokolloidhaltigen Klebeschicht (32) befestigt ist und ein Polyether-Polyurethan-Film ist.

5. Vorrichtung gemäß Anspruch 4, bei der die mit Flüssigkeiten wechselwirkende, hydrokolloidhaltige Klebeschicht (32) etwa 10 mils (254 $\mu$m) dick, die Trägerfilmschicht (34) etwa 1 mils (25,4 $\mu$m) dick, und das temperaturempfindliche Flüssigkristallband (11) etwa 1 bis 15 mils (25,4 bis 381 $\mu$m) dick ist.

6. Vorrichtung gemäß Anspruch 4, bei der die mit Flüssigkeiten wechselwirkende, hydrokolloidhaltige Klebeschicht (32) etwa 20 mils (508 $\mu$m) dick, die Trägerfilmschicht (34) etwa 1 mils (25,4 $\mu$m) dick, und das temperaturempfindliche Flüssigkristallband (11) etwa 1 bis 15 mils (25,4 bis 381 $\mu$m) dick ist.

7. Vorrichtung gemäß Anspruch 3, bei der eine Oberfläche einer polymeren Schaumschicht (33) an der mit Flüssigkeiten wechselwirkenden, hydrokolloidhaltigen Klebeschicht (32) befestigt ist, wobei die Schaumschicht auf der gegenüberliegenden Seite eine Haut- oder Trägerschicht (34) hat.

8. Vorrichtung gemäß Anspruch 7, bei der die mit Flüssigkeiten wechselwirkende, hydrokolloidhaltige Klebeschicht (32) etwa 50 mils (1270 $\mu$m) dick ist, der polymere Schaum (33) ein offenzelliger Polyester-Polyurethan-Schaum mit etwa 60 mils (1524 $\mu$m) Dicke mit einer Polyurethanhaut ist, und das temperaturempfindliche Flüssigkristallband (11) auf der Polyurethanhaut befestigt und etwa 1 bis 15 mils (25,4 bis 381 $\mu$m) dick ist.

9. Vorrichtung gemäß einem der vorhergehenden Ansprüche, bei der das temperaturempfindliche Flüssigkristallband (11) eine Vielzahl von Anzeigen besitzt, die unterschiedliche Temperaturbereiche wiedergeben.

**Revendications**

1. Dispositif médical comprenant un pansement (31) à couche adhésive (32) de contact avec la peau ou la plaie, comprenant un ou plusieurs hydrocolloïdes interactifs avec les liquides, solubles ou gonflant dans l'eau, dispersés dans une substance élastomère, et une couche de pellicule dorsale (34) de même étendue que ladite couche adhésive (32) de contact avec la peau ou la plaie, caractérisé par un indicateur comprenant une bande (11) à cristaux liquides de détection de la température, fixée à au moins une partie de la surface de ladite couche de pellicule dorsale (34) opposée à la couche adhésive (32) de contact avec la peau ou la plaie, de sorte que, quand le dispositif est en place sur le corps, les renseignements concernant l'état de la peau ou de la plaie au-dessous du pansement (31) sont disponibles sans qu'il soit nécessaire d'ôter le pansement (31).

2. Dispositif selon la revendication 1, dans lequel lesdits hydrocolloïdes solubles ou gonflant dans l'eau sont choisis entre la carboxyméthylcellulose sodique, la pectine, la gélatine, le guar, la gomme adragante, le karaya, et leurs mélanges, et ladite substance élastomère est choisie entre le polyisobutylène, les mélanges de polyisobutylènes, et les mélanges d'un ou plusieurs polyisobutylènes et d'un ou plusieurs élastomères choisis entre le caoutchouc butyl et les copolymères radiaux ou séquencés du styrène.

**3.** Dispositif selon la revendication 2, dans lequel ladite couche adhésive (32) contenant des hydrocolloïdes interactifs avec les liquides comprend, en pourcentages pondéraux, environ 8% de polyisobutylène, environ 16,25% de caoutchouc butyl, environ 6% de copolymère séquencé styrène-isoprène-styrène, environ 12,75% d'agent d'adhérence, environ 11,5% d'huile minérale, environ 15% de carboxyméthylcellulose sodique, environ 15% de pectine, environ 15% de gélatine, et environ 0,5% d'anti-oxygène.

**4.** Dispositif selon la revendication 3, dans lequel ladite couche de pellicule dorsale (34) du pansement est fixée directement à ladite couche adhésive (32) contenant des hydrocolloïdes interactifs avec les liquides, et consiste en une pellicule de polyéther polyuréthanne.

**5.** Dispositif selon la revendication 4, dans lequel ladite couche adhésive (32) contenant des hydrocolloïdes interactifs avec les liquides est épaisse d'environ 10 millipouces (254 $\mu$m), ladite couche (34) de pellicule dorsale est épaisse d'environ 1 millipouce (25,4 $\mu$m), et ladite bande (11) à cristaux liquides de détection de la température est épaisse d'environ 1 à 15 millipouces (25,4 à 381 $\mu$m).

**6.** Dispositif selon la revendication 4, dans lequel ladite couche adhésive (32) contenant des hydrocolloïdes interactifs avec les liquides est épaisse d'environ 20 millipouces (508 $\mu$m), ladite couche (34) de pellicule dorsale est épaisse d'environ 1 millipouce (25,4 $\mu$m), et ladite bande (11) à cristaux liquides de détection de la température est épaisse d'environ 1 à 15 millipouces (25,4 à 381 $\mu$m).

**7.** Dispositif selon la revendication 3, dans lequel l'une des surfaces d'une couche de mousse polymère (33) est fixée à ladite couche adhésive (32) contenant des hydrocolloïdes interactifs avec les liquides, ladite couche de mousse portant sur sa surface opposée une couche de peau ou dorsale (34).

**8.** Dispositif selon la revendication 7, dans lequel ladite couche adhésive (32) contenant des hydrocolloïdes interactifs avec les liquides est épaisse d'environ 50 millipouces (1270 $\mu$m), ladite mousse polymère (33) est une mousse de polyester polyuréthanne à cellules ouvertes, épaisse d'environ 60 millipouces (1524 $\mu$m), à peau de polyuréthanne, et ladite bande (11) à cristaux liquides de détection de la température est fixée à ladite peau de polyuréthanne et est épaisse d'environ 1 à 15 millipouces (25,4 à 381 $\mu$m).

**9.** Dispositif selon l'une quelconque des revendications précédentes, dans lequel ladite bande (11) à cristaux liquides de détection de la température porte plusieurs repères représentatifs de différents intervalles de température.

FIG. 1

FIG. 2

FIG.3

11

FIG. 4

FIG. 5

FIG. 6